# EUROPEAN PATENT APPLICATION

(11) **EP 4 624 573 A1**
(43) Date of publication of application: **01.10.2025**
(21) Application number: 22966467.7
(22) Date of filing: 22.11.2022
(51) Int. Cl.: C12N 15/11, C12Q 1/6876

(54) **PROBE/PRIMER LIBRARY FOR CANCER DIAGNOSIS**

(71) Applicant: Quantdetect, Inc., Tokyo 100-0004 (JP)
(72) Inventor: NISHIZUKA, Satoshi, Shiwa-gun, Iwate 028-3694 (JP); HIRAKI, Hayato, Shiwa-gun, Iwate 028-3694 (JP)
(74) Representative: Hoffmann Eitle
(86) International application number: PCT/JP2022/043189
(87) International publication number: WO 2024/111057

(57) **Abstract**

An analytical means quickly applicable to more cancers is provided. A library constituted by a plurality of probes and/or primers for detecting a mutation relevant to a cancer in a gene selected from the group consisting of ARID1A, BRAF, EGFR, FGFR2, FGFR3, HRAS, KRAS, and PIK3CA or a promoter region thereof, wherein the plurality of probes and/or primers include those for detecting c.5965C>T, c.5548dup, and c.3826C>T for ARID1A; c.1799T>A for BRAF; c.2573T>G, c.2235 _2249del, and c.2236_2250del for EGFR; c.755C>G, c.1650T>A, and c.1147T>C for FGFR2; c.746C>G, c.1124A>G, and c.742C>T for FGFR3; c.37G>C, c.182A>G, c.35G>T, c.182A>T, c.181C>A, c.34G>A, and c.35G>A for HRAS; c.35G>A, c.35G>T, c.38G>A, and c.34G>T for KRAS; and c.3140A>G, c.1633G>A, and c.1624G>A for PIK3CA.

## Description

### Technical Field

This invention relates to probes and primers for detecting a mutation in a cancer-related gene, which are useful for early diagnosis of cancer relapse.

### Background Art

Detection of DNAs derived from cancer cells leaked into the peripheral blood, i.e., tumor-derived circulating DNAs (circulating tumor DNAs, ctDNAs), used as diagnosis of cancer relapse is attracting attention. Digital PCR (dPCR) enables counting how many sets of a specific gene sequence were contained in a sample and highly sensitive quantification thereof, and therefore it is suitable for detecting ctDNAs, of which concentrations in samples are considered to be very low.

Like other PCR techniques, dPCR uses primers that determine a range of a gene sequence to be amplified and two types of probes (mutant and wild type) that complementarily bind to parts of the amplified gene sequences. Normally, in order to perform a dPCR test on ctDNA, it is necessary to separately identify a patient-specific gene mutation by analysis using a next-generation sequencer (NGS) or the like, and obtain a set of primers and probes to detect the mutation. In this regard, the inventors of the present invention have conceived an idea of comprehensively preparing in advance a set of primers and probes to detect a mutation in TP53, known as one of the cancer suppressor genes, and have proposed preparation of an OTS (Off-The Shelf, which means ready-to-use) library (Patent documents 1 and 2).

### Prior Art References

### Patent documents

Patent document 1: Japanese Patent No. 6544783
Patent document 2: International Publication WO2020/026776

### Summary of the Invention

### Problems to be solved by the invention

It is desirable to have sets of primers and probes prepared for detecting gene mutations relevant to various cancers so that a wider range of cancers can be immediately addressed when necessary.

It is also desirable that all of such comprehensively prepared sets of primers and probes can be used in digital PCR under similar conditions, or most of the sets can be used in digital PCR under identical conditions.

### Means for solving the problems

The inventors of the present invention recently selected genetic mutations that are found at relatively high frequencies in human cancers on the basis of databases of published documents, The Cancer Genome Atlas (TCGA, https://www.cancer.gov/about-nci/organization/ccg/research/structural-genomics/tcga), Catalogue Of Somatic Mutation In Cancer (COSMIC, https://cancer.sanger.ac.uk/cosmic), and the National Cancer Institute, Center for Cancer Genomics and Advanced Therapeutics, as well as the results of observational studies by the inventors. Then, the inventors of the present invention designed primers and probes for the detection thereof, confirmed the operations thereof, "created a library" thereof that enables quick dPCR analyses in many cancer patients, and thus accomplished the present invention. The present invention provides the followings.
[1] A library constituted by a plurality of probes and/or primers for detecting a mutation relevant to a cancer in a gene selected from the group consisting of ARID1A, BRAF, EGFR, FGFR2, FGFR3, HRAS, KRAS, and PIK3CA or a promoter region thereof, wherein the plurality of probes and/or primers include those for detecting:
   c.5965C>T, c.5548dup, and c.3826C>T for ARID1A;
   c.1799T>A for BRAF;
   c.2573T>G, c.2235_2249del, and c.2236_2250del for EGFR;
   c.755C>G, c.1650T>A, and c.1147T>C for FGFR2;
   c.746C>G, c.1124A>G, and c.742C>T for FGFR3;
   c.37G>C, c.182A>G, c.35G>T, c.182A>T, c.181C>A, c.34G>A, and c.35G>A for HRAS;
   c.35G>A, c.35G>T, c.38G>A, and c.34G>T for KRAS; and
   c.3140A>G, c.1633G>A, and c.1624G>A for PIK3CA.
[2] The library according to 1, which further comprises probes and/or primers for detecting:
   c.5161C>T, c.2077C>T, c.6259G>A, c.4003C>T, c.4336C>T, c.5164C>T, and c.6472C>T for ARID1A,
   c.1798_1799delinsAA, c.1801A>G, c.1406G>C, and c.1798G>A for BRAF, c.2369C>T, c.2240_2257del, c.2240_2254del, c.2582T>A, c.866C>T, c.2156G>C, and c.1793G>T for EGFR,
   c.1127A>G, c.557T>C, c.1650T>G, c.696A>G, c.1978A>G, c.758C>G, and c.870G>C for FGFR2,
   c.1114G>T, c.1954A>G, c.1117A>T, c.417C>T, c.1955A>T, c.2095G>T, and c.1178C>A for FGFR3,
   c.38G>T, c.38G>A, and c.34G>T for HRAS,
   c.35G>C, c.34G>A, c.34G>C, c.37G>T, c.183A>C, and c.182A>T for KRAS, and
   c.3140A>T, c.263G>A, c.1035T>A, c.1636C>A, c.1258T>C, c.1634A>G, and c.2176G>A for PIK3CA.
[3] The library according to 1, which is for detecting a mutation relevant to a cancer by digital PCR, and wherein the primers are used at a final concentration of 450 to 3600 nM.
[4] The library according to 1, which is for detecting a mutation relevant to a cancer by digital PCR, and wherein the digital PCR is performed in the presence of at least one of 20 to 500 µM 7-deaza-2'-deoxy-guanosine-5'-triphosphate and 0.15 to 3.8% dimethyl sulfoxide, as the case may be.
[5] The library according to 1, which is for detecting a mutation relevant to a cancer by digital PCR, and wherein annealing is performed at 55 to 61°C.
[6] The library according to any one of 1 to 5, wherein the probes and/or primers are oligonucleotides that may be labeled, each of which consists of any one of the sequences of SEQ ID NOS: 1 to 144 that may contain a modified base.
[7] A method for analyzing a mutation relevant to a cancer in a gene selected from the group consisting of ARID1A, BRAF, EGFR, FGFR2, FGFR3, HRAS, KRAS, and PIK3CA in a test subject, the method comprising the following steps (1) to (3):
   (1) preparing a library constituted by a plurality of probes and/or primers, wherein the plurality of probes and/or primers include those for detecting mutations of:
      c.5965C>T, c.5548dup, and c.3826C>T for ARID1A;
      c.1799T>A for BRAF;
      c.2573T>G, c.2235_2249del, and c.2236_2250del for EGFR;
      c.755C>G, c.1650T>A, and c.1147T>C for FGFR2;
      c.746C>G, c.1124A>G, and c.742C>T for FGFR3;
      c.37G>C, c.182A>G, c.35G>T, c.182A>T, c.181C>A, c.34G>A, and c.35G>A for HRAS;
      c.35G>A, c.35G>T, c.38G>A, and c.34G>T for KRAS; and
      c.3140A>G, c.1633G>A, and c.1624G>A for PIK3CA.
   (2) selecting a set of probes and/or primers for detecting one mutation from the prepared library; and
   (3) analyzing a mutation in a nucleic acid obtained from the test subject by digital PCR using the selected set of probes and/or primers.
[8] The method according to 7, wherein the primers are used at a final concentration of 450 to 3600 nM.
[9] The method according to 7, wherein the digital PCR is performed in the presence of at least one of 20 to 500 nM 7-deaza-2'-deoxy-guanosine-5'-triphosphate and 0.15 to 3.8% dimethyl sulfoxide, as the case may be.
[10] The method according to 7, wherein annealing is performed at 55 to 61°C.
[11] The method according to any one of 7 to 10, wherein the probes and/or primers are oligonucleotides that may be labeled, each of which consists of any one of the sequences of SEQ ID NOS: 1 to 137 that may contain a modified base.
[12] A probe or primer consisting of any of the following sequences (lowercase a is 2-amino-deoxyadenine and lowercase c is 5-methyl-deoxycytosine), which is for detecting a mutation relevant to a cancer by digital PCR. 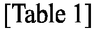 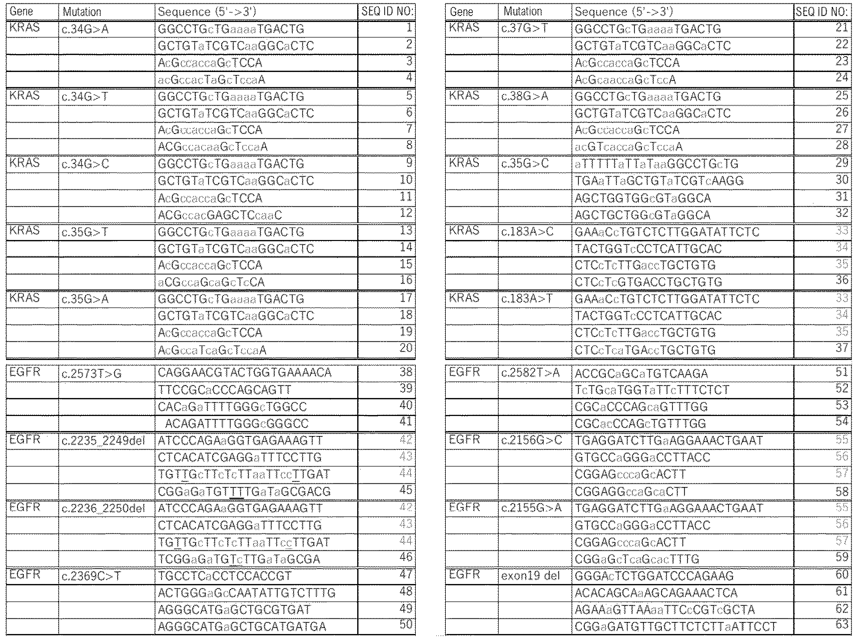 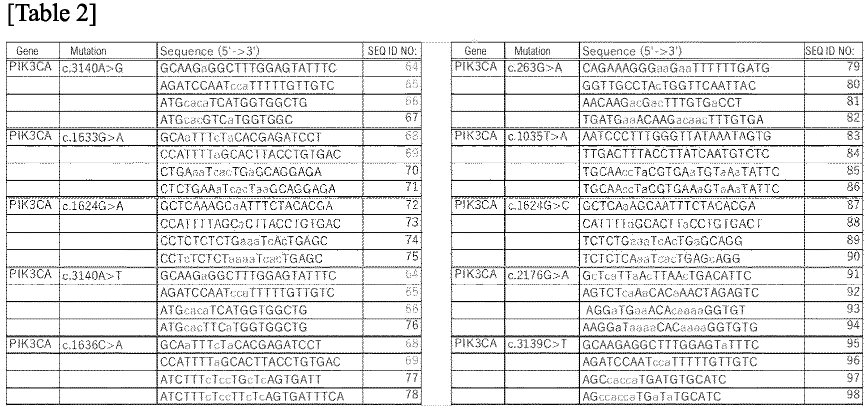 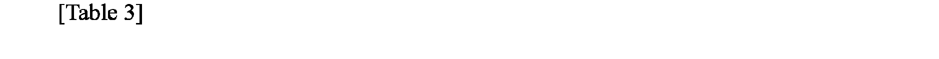 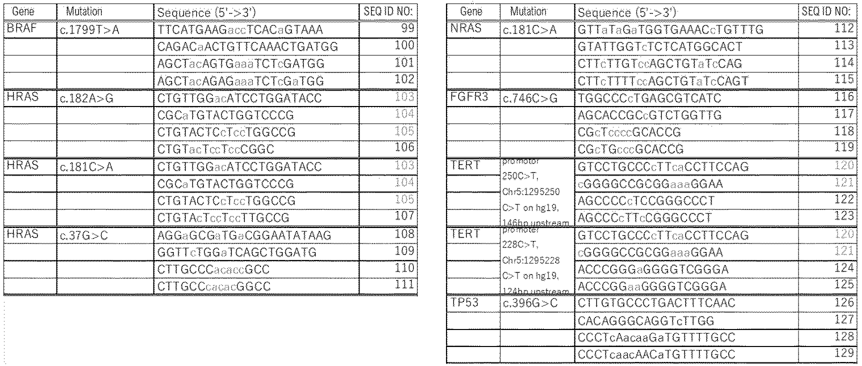
[13] A library for use in digital PCR comprising at least one set of oligonucleotides selected from the group consisting of the following sets of oligonucleotides, wherein sequences of the oligonucleotides may contain a modified nucleotide, and the oligonucleotides may be labeled:
   oligonucleotides consisting of the sequences of SEQ ID NOS: 1 to 4,
   oligonucleotides consisting of the sequences of SEQ ID NOS: 5 to 8,
   oligonucleotides consisting of the sequences of SEQ ID NOS: 9 to 12,
   oligonucleotides consisting of the sequences of SEQ ID NOS: 13 to 16,
   oligonucleotides consisting of the sequences of SEQ ID NOS: 17 to 20,
   oligonucleotides consisting of the sequences of SEQ ID NOS: 21 to 24,
   oligonucleotides consisting of the sequences of SEQ ID NOS: 25 to 28,
   oligonucleotides consisting of the sequences of SEQ ID NOS: 29 to 32,
   oligonucleotides consisting of the sequences of SEQ ID NOS: 33 to 36,
   oligonucleotides consisting of the sequences of SEQ ID NOS: 33 to 35 and 37,
   oligonucleotides consisting of the sequences of SEQ ID NOS: 38 to 41,
   oligonucleotides consisting of the sequences of SEQ ID NOS: 42 to 45,
   oligonucleotides consisting of the sequences of SEQ ID NOS: 42 to 44 and 46,
   oligonucleotides consisting of the sequences of SEQ ID NOS: 47 to 50,
   oligonucleotides consisting of the sequences of SEQ ID NOS: 51 to 54,
   oligonucleotides consisting of the sequences of SEQ ID NOS: 55 to 59,
   oligonucleotides consisting of the sequences of SEQ ID NOS: 60 to 63,
   oligonucleotides consisting of the sequences of SEQ ID NOS: 64 to 67,
   oligonucleotides consisting of the sequences of SEQ ID NOS: 68 to 71,
   oligonucleotides consisting of the sequences of SEQ ID NOS: 72 to 75,
   oligonucleotides consisting of the sequences of SEQ ID NOS: 64 to 66, and 76,
   oligonucleotides consisting of the sequences of SEQ ID NOS: 68, 69, 77, and 78,
   oligonucleotides consisting of the sequences of SEQ ID NOS: 79 to 82,
   oligonucleotides consisting of the sequences of SEQ ID NOS: 83 to 86,
   oligonucleotides consisting of the sequences of SEQ ID NOS: 87 to 90,
   oligonucleotides consisting of the sequences of SEQ ID NOS: 91 to 94,
   oligonucleotides consisting of the sequences of SEQ ID NOS: 95 to 98,
   oligonucleotides consisting of the sequences of SEQ ID NOS: 99 to 102,
   oligonucleotides consisting of the sequences of SEQ ID NOS: 103 to 106,
   oligonucleotides consisting of the sequences of SEQ ID NOS: 103 to 105, and 107,
   oligonucleotides consisting of the sequences of SEQ ID NOS: 108 to 111,
   oligonucleotides consisting of the sequences of SEQ ID NOS: 112 to 115,
   oligonucleotides consisting of the sequences of SEQ ID NOS: 116 to 119,
   oligonucleotides consisting of the sequences of SEQ ID NOS: 120 to 123,
   oligonucleotides consisting of the sequences of SEQ ID NOS: 120, 121, 124, and 125, and
   oligonucleotides consisting of the sequences of SEQ ID NOS: 126 to 129.

### Effects of the Invention

The present invention enables immediate response to more cancers when needed.

The present invention enables early detection of relapse of various cancers after treatment thereof.

### Brief Description of the Drawings

[Fig. 1] Differences in results of digital PCR depending on the primer concentration.
[Fig. 2] Differences in results of digital PCR depending on the annealing temperature.
[Fig. 3] Differences in results of digital PCR depending on the concentration of 7-deaza-2'-deoxy-guanosine-5'-triphosphate (7-deaza-dGTP) added.

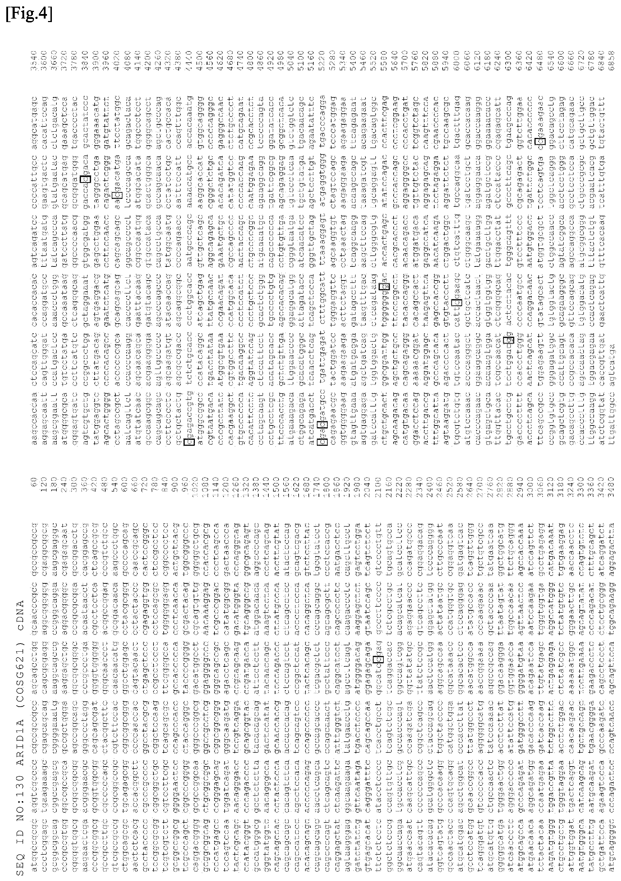

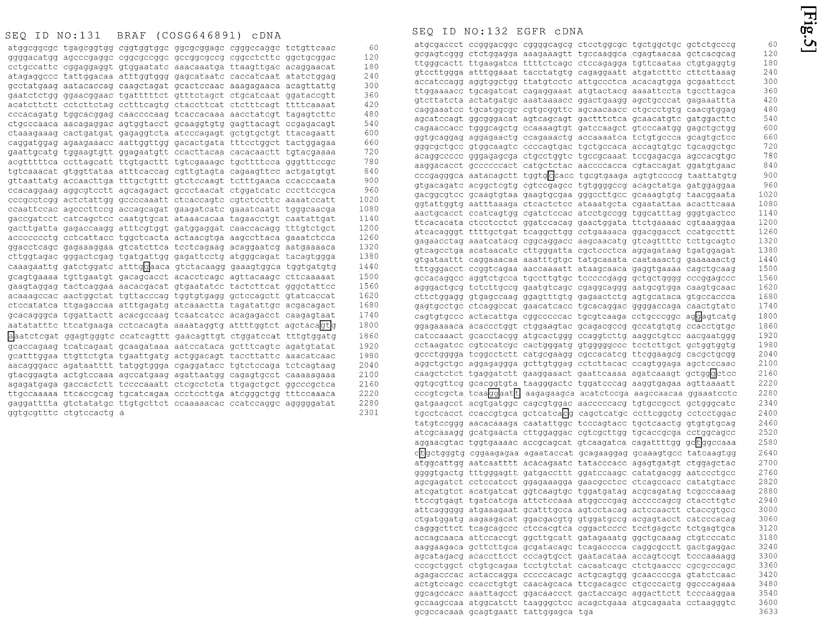

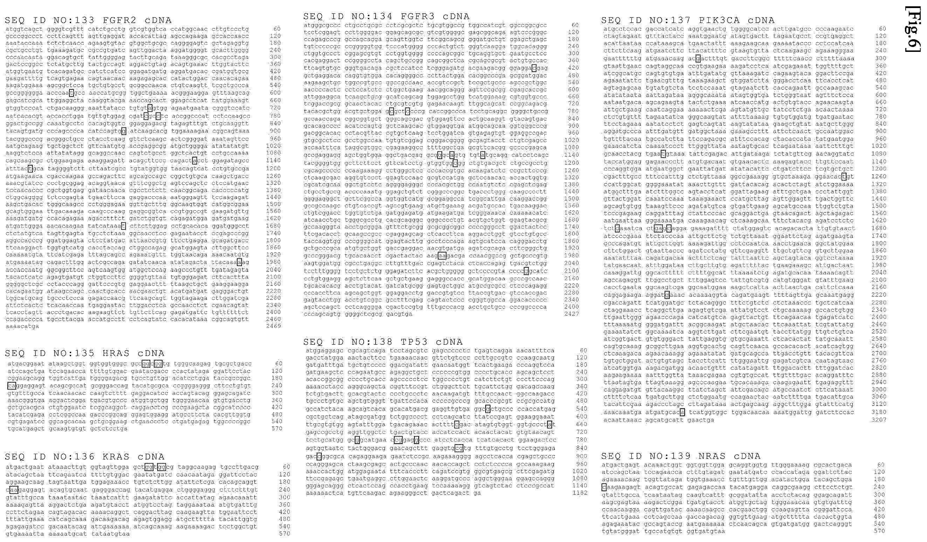

[Fig. 4] SEQ ID NO: 130 ARID1A (COSG621) cDNA. The bases at positions relevant to the present invention are indicated with boxes (the same shall apply to Figs. 5 and 6).
[Fig. 5] SEQ ID NO: 131 BRAF (COSG646891) cDNA, and SEQ ID NO: 132 EGFR cDNA.
[Fig. 6] SEQ ID NO: 133 FGFR2 cDNA, SEQ ID NO: 134 FGFR3 cDNA, SEQ ID NO: 135 HRAS cDNA, SEQ ID NO: 136 KRAS cDNA, SEQ ID NO: 137 PIK3CA cDNA, SEQ ID NO: 138 TP53 cDNA, and SEQ ID NO: 139 NRAS cDNA.

### Modes for Carrying out the Invention

The present invention provides a library comprising probes and/or primers for detecting mutations associated with cancers. With respect to the present invention, the terms probe and/or primer mean at least one of probe and primer, unless especially stated. That is, "probe and/or primer" means only probe, only primer, or probe and primer.

### [Library]

The library of the present invention is a collection of probes and/or primers for use in PCR for detecting mutations in genes relevant to cancers or promoter regions thereof. The library is typically a collection of probes and/or primers for detecting each mutation, which are stored in separate containers.

### (Genes and mutations associated with the library)

Examples of the genes relevant to cancers are listed in the following table. The library can be one for detecting any mutation in any one or more of these genes relevant to cancers.

**[Table 4]**

| | | | | | | | | |
|---|---|---|---|---|---|---|---|---|
| *AKT1* | *CALR* | *ERBB3* | *FOXL2* | *JAK3* | *MSH6* | *PIK3CB* | *RB1* | *STAG2* |
| *ALK* | *CARD11* | *ERBB4* | *FOXP1* | *KDR* | *MTOR* | *PIK3CD* | *RET* | *STAT3* |
| *ARID1A* | *CD79B* | *ERCC2* | *GNA11* | *KIT* | *MYC* | *PIK3CG* | *RHOA* | *STK11* |
| *ARID2* | *CDH1* | *ERCC5* | *GNAQ* | *KRAS* | *MYCN* | *PIK3R1* | *RNF43* | *TERT* |
| *ASXL1* | *CDKN2A* | *ESR1* | *GNAS* | *MAP2K1* | *MYD88* | *PIK3R2* | *ROS1* | *TET2* |
| *ATM* | *CHD4* | *EZH2* | *H3F3A* | *MAPK1* | *NF2* | *PMS2* | *RXRA* | *TP53* |
| *ATRX* | *CIC* | *FANCA* | *H3F3B* | *MAX* | *NFE2L2* | *POLE* | *SF3B1* | *VHL* |
| *BCL6* | *CTNNB1* | *FBXW7* | *HRAS* | *MED12* | *NOTCH1* | *PPP2R1A* | *SMAD2* | *WT1* |
| *BCOR* | *DICER1* | *FGFR2* | *IDH1* | *MET* | *NRAS* | *PTEN* | *SMAD4* | *XPO1* |
| *BRAF* | *DNMT3A* | *FGFR3* | *IDH2* | *MLH1* | *NT5C2* | *PTPN11* | *SMARCA4* | |
| *BRCA1* | *EGFR* | *FLT3* | *IRF4* | *MPL* | *PBRM1* | *RAC1* | *SPOP* | |
| *BRCA2* | *ERBB2* | *FOXA1* | *JAK2* | *MSH2* | *PIK3CA* | *RAF1* | *SRSF2* | |

In a preferred embodiment, the library is for detecting mutations in ARID1A, BRAF, EGFR, FGFR2, FGFR3, HRAS, KRAS, and PIK3CA, among thees genes.

In another preferred embodiment, the library is for detecting mutations in ARID1A, BRAF, EGFR, FGFR2, FGFR3, HRAS, KRAS, and PIK3CA mentioned above as well as NRAS, and TERT.

In another preferred embodiment, the library is for detecting mutations in ARID1A, BRAF, EGFR, FGFR2, FGFR3, HRAS, KRAS, and PIK3CA, or ARID1A, BRAF, EGFR, FGFR2, FGFR3, HRAS, KRAS, PIK3CA, NRAS, and TERT mentioned above as well as TP53.

The library is preferably a collection that enables detection of mutations at 25 or more positions, more preferably a collection that enables detection of mutations at 50 or more positions, still more preferably a collection that enables detection of mutations at 166 or more positions, further preferably a collection that enables detection of mutations at 1000 or more positions.

The library comprises a plurality of oligonucleotides to detect mutations at plurality of positions, and can be used in various different ways. Typically, it is used to analyze a target mutation (one position) for a single test subject. For example, it may be used for a certain patient to analyze whether a mutation detected in a primary tumor before a treatment is detected in the blood during the process of the treatment, or whether the mutation is detected in the blood after the treatment for the purpose of diagnosing relapse. The library may also be used to analyze mutations at a plurality of positions in a single subject. The library may also be used to analyze a target mutation (one position) in a plurality of test subjects, mutations at a plurality of positions in each of a plurality of test subjects, and so forth.

The term mutation referred to in the explanations of the present invention means a mutation of base (also expressed as nucleotide), unless especially noted. Mutation of a certain base may be substitution of another base, deletion (del), duplication (dup), and so forth.

For the present invention, mutations are referred to according the standard rules well known to those skilled in the art (https://varnomen.hgvs.org/recommendations/DNA/variant/duplication/, Hum Mutat 2016; 37:564-569). For the present invention, position of the mutation is described according to the information provided in the COSMIC database and the information described in Sequence Listing, unless especially noted.

The library may consist only of oligonucleotides that can function as primers, only of oligonucleotides that can function as probes, or both. A mutation is usually detected by amplifying a portion in a nucleic acid that may contain the target mutation by polymerase chain reaction (PCR) using primers, and detecting the presence of the target mutation in the amplified product using a probe. Therefore, the library preferably comprises a probe and primer pair for the target mutation as a set. The term amplification product means DNAs amplified by PCR in the case of PCR, and may also be called amplicon.

The probe included in the library may also be one for detecting a sequence not having the target mutation (wild type), or may be one for detecting a sequence having the target mutation (mutant). The library may include both types of probes.

The primer included in the library is for amplifying a portion including the position of the target mutation in a nucleic acid contained in a sample, and it is usually preferably included in the library as a pair of forward primer corresponding to a plus strand, and reverse primer corresponding to a minus strand.

The library is for detecting mutations relevant to a cancer, and in one embodiment, it may be one capable of non-intermittently detecting mutations that can occur in a target gene. For example, with respect to TP53, a library that enables non-intermittent detection of mutations in the coding region for 195 amino acids, where a lot of mutations relevant to a cancer are found, specifically includes a set of 1755 primers and probes (see Patent documents 1 and 2).

In another embodiment, the library comprises primer and probe sets for mutations that can occur in the target gene or promoter region thereof and appear with high frequency. According to the study of the inventors of the present invention, the frequency of occurrence (reported) greatly varies depending on the type of the mutation, and therefore the library that targets mutations with higher frequency enables more comprehensive detection of mutations.

According to the study of the inventors of the present invention, for TP53, for example, if mutations of an appearance frequency rank not lower than 10th place are targeted, a library that enables detection of at least 30% of TP53 mutations can be designed. Further, if mutations of an appearance frequency rank not lower than 40th place are targeted, a library that enables detection of at least 50% of TP53 mutations can be designed. Further, if mutations of an appearance frequency rank not lower than 232nd place are targeted, a library that enables detection of at least 80% of TP53 mutations can be designed (see Patents 1 and 2).

Therefore, one particularly preferred library is one that is designed so that it enables detection of at least one type of mutation, e.g., 2 or more types, preferably 4 or more types, more preferably 6 or more types, still more preferably 10 or more types, further preferably 20 or more types, of mutations from those of higher frequencies (those ranked at the highest places in each number of the types from the highest in the ranking of mutations in frequency).

Mutations that are preferably used as the detection target are listed in the following table in the order of frequency of appearance (Fraction (%) in the following table) from the highest for each gene.

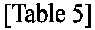

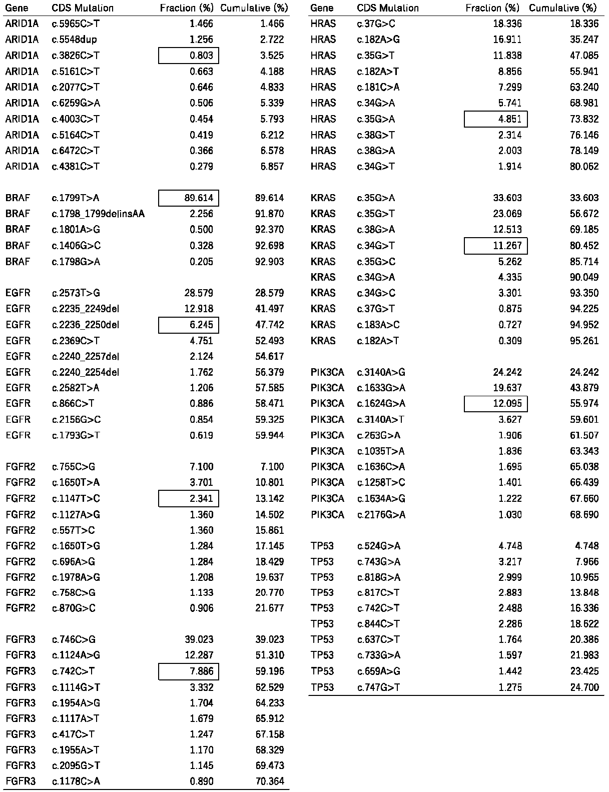

In the table, the values of Fraction (%) with a large difference from that of the next rank are indicated with boxes.

Therefore, in a preferred embodiment, the library is constituted by a plurality of probes and/or primers for detecting the mutations mentioned in the above table with the frequencies of appearance indicated with boxes and those with further higher frequencies of appearance.

More specifically, in one embodiment, the library is a library that is constituted by a plurality of probes and/or primers for detecting a mutation relevant to a cancer in a gene selected from the group consisting of ARID1A, BRAF, EGFR, FGFR2, FGFR3, HRAS, NRAS, KRAS, and PIK3CA or a promoter region thereof, wherein the plurality of probes and/or primers comprise those for detecting:
c.5965C>T, c.5548del, and c.5548dup for ARID1A;
c.1799T>A for BRAF;
c.2573T>G, c.2235_2249del, and c.2236_2250del for EGFR;
c.755C>G, c.1650T>A, and c.1147T>C for FGFR2;
c.746C>G, c.1124A>G, and c.742C>T for FGFR3;
c.37G>C, c.182A>G, c.35G>T, c.182A>T, c.181C>A, c.34G>A, and c.35G>A for HRAS;
c.35G>A, c.35G>T, c.38G>A, and c.34G>T for KRAS; and
c.3140A>G, c.1633G>A, and c.1624G>A for PIK3CA.

Preferably, the library is a library that is constituted by a plurality of probes and/or primers for detecting a mutation relevant to a cancer in a gene selected from the group consisting of ARID1A, BRAF, EGFR, FGFR2, FGFR3, HRAS, NRAS, KRAS, and PIK3CA or a promoter region thereof, wherein the plurality of probes and/or primers are those for detecting:
c.5965C>T, c.5548dup, c.3826C>T, c.5161C>T, c.2077 C>T, c.4003C>T, c.5164C>T, c.6259G>A, c.6472C>T, and c.4381C>T forARID1A,
c.1799T>A, c.1798_1799delinsAA, c.1801A>G, c.1406G>C, and c.1798G>A for BRAF,
c.2573T>G, c.2235_2249del, c.2236_2250del, c.2369C>T, c.2240_2257del, c.2240 _2254del, c.2582T>A, c.866C>T, c.2156G>C, and c.1793G>T for EGFR
c.755C>G, c.1650T>A, c.1147T>C, c.1127A>G, c.557T>C, c.1650T>G, c.696A>G, c.1978A>G, c.758C>G, and c.870G>C for FGFR2,
c.746C>G, c.1124A>G, c.742C>T, c.1114G>T, c.1954A>G, c.1117A>T, c.417C>T, c.1955A>T, c.2095G>T, and c.1178C>A for FGFR3,
c.37G>C, c.182A>G, c.35G>T, c.182A>T, c.181C>A, c.34G>A, c.35G>A, c.38G>T, c.38G>A, and c.34G>T for HRAS,
c.35G>A, c.35G>T, c.38G>A, c.34G>T, c.35G>C, c.34G>A, c.34G>C, c.37G>T, c.183A>C, and c.182A>T for KRAS, and
c.3140A>G, c.1633G>A, c.1624G>A, c.3140A>T, c.263G>A, c.1035T>A, c.1636C>A, c.1258T>C, c.1634A>G, and c.2176G>A for PIK3CA.

These libraries may comprise probes and/or primers for detecting mutations relevant to a cancer in NRAS. Examples of the mutations relevant to a cancer in NRAS include the following:
c.181C>A p.Q61K.

These libraries may comprise probes and/or primers for detecting mutations relevant to a cancer in TERT or a promoter region thereof. Examples of the mutations relevant to a cancer in TERT or a promoter region thereof include the followings:
TERT promoter 250C>T, Chr5:1295250 C>T on hg19, 146bp upstream of the translation start, and
TERT promoter 228C>T, Chr5:1295228 C>T on hg19, 124bp upstream of the translation start.

These libraries may also comprise probes and/or primers for detecting mutations relevant to a cancer in TP53. Details of the probes and/or primers for detecting mutations relevant to a cancer in TP53 are described in Patent documents 1 and 2 mentioned above.

### (Design of probe and/or primer)

Although the probes and/or primers included in the library can be variously designed so that they enable analysis of mutations in nucleic acids contained in a sample extracted from a test subject, they are preferably designed so that they enable analysis of ctDNAs existing in blood of a test subject in free forms. This is because, if ctDNAs in blood can be analyzed, mutant DNA characteristic to a tumor can be systemically traced. That is also because such analysis can be easily performed for a long period of time with less burdens on the patient after the treatment, and can be expected to enable quick transfer to secondary treatment based on quick monitoring of increase in "tumor burden" after the treatment. The analysis includes detection, quantification, and so forth. The test subject may be a cancer patient, a person suspected to have a cancer, or the like.

From the viewpoint of analysis of ctDNAs, the probes and/or primers included in the library are designed so that they enable analysis of a mutant (also referred to as mutant sequence) of a small ratio in the presence of a large excess amount of wild type (also referred to as non-mutated sequence or reference sequence). This is because, in the cancer diagnoses, blood concentration of ctDNA (existence ratio of mutated sequence relative to non-mutated sequence, also referred to as allele frequency) is 1% or lower in many cases, and therefore it is desirable that highly sensitive analysis can be conducted. An example of such a highly sensitive analysis method is dPCR.

dPCR (Vogelstein and Kinzler, Proc. Natl. Acad. USA, 96, 9236-9241, 1999) is a developed form of the conventional PCR method, and it enables direct quantification of a target nucleic acid. dPCR used in combination with a concentration procedure is suitable for detecting an extremely small amount of mutations. Since a DNA sample is diluted even to a single reaction level in dPCR, the starting material should be either a wild type or mutant in each PCR. The concentration is carried out from a single molecule, and thousands of concentration reactions are simultaneously performed in parallel. A large number of PCRs are performed from the same starting material, and then mutated molecules are isolated and detected. By performing quantification for chambers containing the final product of PCR after the PCR amplification, absolute quantity of the nucleic acid can be calculated. Various dPCR-based systems usable for the present invention are marketed. The fundamental methodology of dPCR is described in, for example, Sykes et al., Biotechniques, 13(3):444-449, 1992.

From the viewpoint of analysis of ctDNA, the probes and/or primers are preferably designed so that they enable amplification of a comparatively short nucleic acid fragment as a target sequence, and detection of the target mutation. The size of amplification product in usual PCR is 100 to 150 bp, and reduction of sensitivity and accuracy may cause a problem. It is considered that, in order to improve the analysis performance, it is important to shorten the size of amplification product as much as possible (Antonov J, et al., Lab. Invest., 2005 Aug;85(8):1040-50; Kong H, et al., Sci. Rep., 2014 Nov. 28;4:7246; and Florent Mouliere et al., PLOS ONE, September 2011, Volume 6, Issue 9, e23418).

For example, there have been developed methods for conducting the analysis with a comparatively short amplification product, for example, an amplification product of 70 bp or shorter, by the means of inclusion of a modified nucleotide in oligonucleotides as primers or probes, or the like. Primers and probes for such methods are known as Hypercool Primer & Probe^{™} (Nihon Gene Research Laboratories, Inc., 1-5-2, Nakano, Miyagino-ku, Sendai-shi, Japan), and a method for designing them is publicized (http://ngrl.co.jp/wp/wp-content/uploads/2015/01/eb3607d6cleb4174f10bb3bde67844fe.pdf). As for detection of mutated sequences of a low ratio in the presence of a large excessive amount of non-mutated sequence, for example, Japanese Patent Unexamined Publication (KOHYO) No. 2010-535031 can be referred to. This technique is based on a treatment protocol for a reaction mixture performed at a temperature lower than the critical denaturation temperature or melting temperature Tm of non-mutated sequence.

According to one of the preferred embodiments, the oligonucleotides included in the library contain a modified nucleotide (also referred to as modified base). Examples of such a modified nucleotide include a diaminopurine analogue (for example, 2'-O-methyl-2,6-diaminopurine), uracil, peptide nucleic acid analogue, biotinmodified analogue, fluorophore-modified analogue, inosine, 7-deazaguanine, 2'-deoxy-2'-fluoro-β-D-arabinonucleic acid (2'F-ANA) nucleotide, locked nucleic acid (LNA), 2'-O,4'-C-ethylene crosslinked nucleic acid (ENA), and so forth. These modifications can increase the difference between Tms of matched or mismatched bases, and enable highly precise analysis.

Particularly preferred examples of the modified nucleotide are 2-amino-deoxyadenine (2aA) and 5-methyl-deoxycytidine (5mC).

The oligonucleotides included in the library can contain mismatched bases or unmatched bases. Those skilled in the art of nucleic acids can determine stability of a double strand in consideration of many variables such as length of oligonucleotide, base composition and sequence of oligonucleotide, ionic strength, and content of mismatch bases. The stability of nucleic acid double strand is represented by the melting temperature (also referred to as denaturation temperature) Tm.

The introduction of a locked nucleic acid into an oligonucleotide increases the affinity of the complementary sequence and increases the melting temperature by several steps (Braasch, D.A. and D.R. Corey, Chem. Biol. (2001), 8:1-7). Locked nucleic acids refer to a class of stereostructurally-restricted nucleotide analogues (see, for example, WO99/14226, Koshkin, A.A., et al., Tetrahedron (1998), 54: 3607-3630, and Obika, S., et al. Tetrahedron (1998), 39: 5401-5404).

The probes included in the library may be modified around the ends thereof so that analysis becomes easy. A fluorescent substance can be used for the modification. Examples of the fluorescent substance include 6FAM (also referred to simply as FAM), HEX, Tide Fluor^{™} 1, ATTO 390, ATTO 425, LC (registered trademark) 480Cyan500, ATTO 465, ATTO 488, Tide Fluor^{™} 2, ATTO 495, ATTO 514, ATTO 520, TET, JOE, CAL Fluor 540, Yakima Yellow, ATTO 532, ATTO Rho6G, LC (registered trademark) Yellow555, CAL Fluor 560, ATTO 542, Quasar (registered trademark) 570, Cy3, ATTO 550, TAMRA, Tide Fluor^{™} 3, ATTO 565, ATTO Rho3B, ROX, ATTO Rho11, ATTO Rho12, Cy3.5, ATTO Thio12, ATTO Rho101, LC (registered trademark) Red610, CAL Fluor 610, Tide Fluor^{™} 4, ATTO 590, TexasRed (SR101), ATTO 594, ATTO Rho13, ATTO 610, LC (registered trademark) Red670, ATTO 620, LC (registered trademark) Red640, ATTO Rho14, ATTO 633, ATTO 647, ATTO 647N, Quasar (registered trademark) 670, Tide Fluor^{™} 5, Cy5, ATTO 655, ATTO Oxa12, ATTO 665, Tide Fluor^{™} 6, Cy5.5, ATTO 680, LC (registered trademark) Red705, Quasar (registered trademark) 705, ATTO 700, ATTO 725, ATTO740, Tide Fluor^{™} 7, and Tide Fluor^{™} 8. Use of different fluorescent substances for a probe for wild type and probe for mutant enables simultaneous analysis of them.

The probes included in the library may also be modified with a quenching substance (also referred to as quencher). Examples of the quenching substance include Dabcyl, BHQ-0 (registered trademark), BHQ-1 (registered trademark), BHQ-2 (registered trademark), BHQ-3 (registered trademark), TQ^{™}1, TQ^{™}2, TQ^{™}3, TQ^{™}4, TQ^{™}5, TQ^{™}6, TQ^{™}7, TAMRA, ATTO 540Q, ATTO 575Q, ATTO 580Q, ATTO 612Q, and BBQ-650 (registered trademark).

### (Specific examples of probes and/or primers)

In one embodiment, the library is a library comprising at least one set of oligonucleotides selected from the group consisting of the following sets:
oligonucleotides consisting of the sequences of SEQ ID NOS: 1 to 4 for detecting KRAS c.34G>A p.G12S,
oligonucleotides consisting of the sequences of SEQ ID NOS: 5 to 8 for detecting KRAS c.34G>T p.G12C,
oligonucleotides consisting of the sequences of SEQ ID NOS: 9 to 12 for detecting KRAS c.34G>C p.G12R,
oligonucleotides consisting of the sequences of SEQ ID NOS: 13 to 16 for detecting KRAS c.35G>T p.G12V,
oligonucleotides consisting of the sequences of SEQ ID NOS: 17 to 20 for detecting KRAS c.35G>A p.G12D,
oligonucleotides consisting of the sequences of SEQ ID NOS: 21 to 24 for detecting KRAS c.37G>T p.G13C,
oligonucleotides consisting of the sequences of SEQ ID NOS: 25 to 28 for detecting KRAS c.38G>A p.G13D,
oligonucleotides consisting of the sequences of SEQ ID NOS:29 to 32 for detecting KRAS c.35G>C p.G12A,
oligonucleotides consisting of the sequences of SEQ ID NOS: 33 to 36 for detecting KRAS c.183A>C p.Q61H,
oligonucleotides consisting of the sequences of SEQ ID NOS: 33 to 35, and 37 for detecting KRAS c.183A>T p.Q61H,
oligonucleotides consisting of the sequences of SEQ ID NOS:38 to 41 for detecting EGFR c.2573T>G p.L858R,
oligonucleotides consisting of the sequences of SEQ ID NOS: 42 to 45 for detecting EGFR c.2235_2249del(GGAATTAAGAGAAGC) p.E746 _A750del,
oligonucleotides consisting of the sequences of SEQ ID NOS:42 to 44, and 46 for detecting EGFR c.2236_2250del(GAATTAAGAGAAGCA) p.E746_A750del,
oligonucleotides consisting of the sequences of SEQ ID NOS:47 to 50 for detecting EGFR c.2369C>T p.T790M,
oligonucleotides consisting of the sequences of SEQ ID NOS: 51 to 54 for detecting EGFR c.2582T>A p.L861Q,
oligonucleotides consisting of the sequences of SEQ ID NOS: 55 to 57, and 59 for detecting EGFR c.2156G>C p.G719A,
oligonucleotides consisting of the sequences of SEQ ID NOS:55 to 57, and 59 for detecting EGFR c.2155G>A p.G719S,
oligonucleotides consisting of the sequences of SEQ ID NOS: 60 to 63 for detecting EGFR exon19 del *Hotspot (a.a. 745-753),
oligonucleotides consisting of the sequences of SEQ ID NOS: 64 to 67 for detecting PIK3CA c.3140A>G p.H1047R,
oligonucleotides consisting of the sequences of SEQ ID NOS: 68 to 71 for detecting PIK3CA c.1633G>A p.E545K,
oligonucleotides consisting of the sequences of SEQ ID NOS: 72 to 75 for detecting PIK3CA c.1624G>A p.E542K,
oligonucleotides consisting of the sequences of SEQ ID NOS: 64 to 66, and 76 for detecting PIK3CA c.3140A>T p.H1047L,
oligonucleotides consisting of the sequences of SEQ ID NOS: 68, 69, 77, and 78 for detecting PIK3CA c.1636C>A p.Q546K,
oligonucleotides consisting of the sequences of SEQ ID NOS: 79 to 82 for detecting PIK3CA c.263G>A p.R88Q,
oligonucleotides consisting of the sequences of SEQ ID NOS: 83 to 86 for detecting PIK3CA c.1035T>A p.N345K,
oligonucleotides consisting of the sequences of SEQ ID NOS: 87 to 90 for detecting PIK3CA c.1624G>C p.E542Q,
oligonucleotides consisting of the sequences of SEQ ID NOS: 91 to 94 for detecting PIK3CA c.2176G>A p.E726K,
oligonucleotides consisting of the sequences of SEQ ID NOS: 95 to 98 for detecting PIK3CA c.3139C>T p.H1047Y,
oligonucleotides consisting of the sequences of SEQ ID NOS: 99 to 102 for detecting BRAF c.1799T>A p.V600E,
oligonucleotides consisting of the sequences of SEQ ID NOS: 103 to 106 for detecting HRAS c.182A>G p.Q61R,
oligonucleotides consisting of the sequences of SEQ ID NOS: 103 to 105, and 107 for detecting HRAS c.181C>A p.Q61K,
oligonucleotides consisting of the sequences of SEQ ID NOS: 108 to 111 for detecting HRAS c.37G>C p.G13R,
oligonucleotides consisting of the sequences of SEQ ID NOS: 112 to 115 for detecting NRAS c.181C>A p.Q61K,
oligonucleotides consisting of the sequences of SEQ ID NOS: 116 to 119 for detecting FGFR3 c.746C>G p.S249C,
oligonucleotides consisting of the sequences of SEQ ID NOS:120 to 123 for detecting TERT promoter 250C>T, Chr5:1295250 C>T on hg19, 146bp upstream of the translation start,
oligonucleotides consisting of the sequences of SEQ ID NOS:120, 121, 124, and 125 for detecting TERT promoter 228C>T, Chr5:1295228 C>T on hg19, 124bp upstream of the translation start, and
oligonucleotides consisting of the sequences of SEQ ID NOS:126 to 129 for detecting TP53 c.396G>C/

Adenine and cytosine contained in the probe and primer sequences may be modified. Specific examples of the primer and probe containing modified bases are listed in the section of Examples described in this specification.

The primers and probes described above can be preferably used for digital PCR (dPCR). dPCR can be carried out in the same scale as that of the conventional dPCR method. Although amounts of nucleic acids, primers and probes to be used in dPCR can be appropriately determined by those skilled in the art, the nucleic acids, primers, and probes can be used in an amount in the ranges of, for example, 0.5 to 50 ng, 0.02 to 2.0 µM, and 0.01 to 1.0 µM, respectively, per one chip of dPCR. Therefore, if the library is constituted as a collection of probes and/or primers contained in tubes, in which the probes and/or primers for detecting mutations are stored in separate containers, e.g., tubes, the typical concentration of the primer in each tube may be 450 to 3600 nM, preferably 500 to 2000 nM, more preferably 600 to 1200 nM, for example, 900 nM, and the same of the probe may be 10 to 1000 nM, preferably 50 to 500 nM, more preferably 100 to 300 nM, for example, 250 nM. When referring to concentrations of primers or probes for the present invention, they are described as the concentration of one type of primer or one type of probe, unless especially stated.

For dPCR, the composition of the system can contain four kinds of dNTPs, buffering agent, salt, surfactant, and enzymes that catalyze the chain extension reaction, in addition to the nucleic acids, primers, and probes in such amounts as described above, as apparent to those skilled in the art.

### (Special notes on dPCR)

According to the study of the inventors of the present invention, primers designed in accordance with the present invention, embodiments thereof, and specific examples thereof are preferably used in dPCR at a final concentration of 450 to 3600 nM. For most of the primers, an example of preferred concentration is 900 nM.

In some cases, however, the reaction of PCR may not proceed efficiently at such concentrations. In such cases, the reaction of PCR can be properly advanced by using primers at higher concentrations. In such cases, specifically, primers should be used at a concentration of 1000 to 3600 nM, preferably 1200 to 3000 nM, more preferably 1500 to 2100 nM, for example, 1800 nM.

In dPCR, when targeting regions with high GC contents, the reaction of PCR may be difficult to proceed. For example, the TERT promoter region has a high GC content, and may be difficult to be amplified as it is with primers. In such cases, the annealing temperature in dPCR can be adjusted to the same level as in the normal cases by adding 7-deaza-2'-deoxy-guanosine-5'-triphosphate (7-deaza-dGTP) or dimethyl sulfoxide (DMSO) to the system.

Specifically, when 7-deaza-dGTP is used, it should be added to the dPCR system at a concentration of 20 to 500 µM, preferably 50 to 200 µM, more preferably 75 to 150 µM, for example, 100 µM. Alternatively, when DMSO is used, it should be added to the dPCR system at a concentration of 0.15 to 3.8%, preferably 0.30 to 2.4%, more preferably 0.50 to 1.0%, for example, 0.75%.

According to the study of the inventors of the present invention, in dPCR using primer and probe sets designed in accordance with the present invention, embodiments thereof, and examples thereof, annealing is performed at 55 to 61°C. More specifically, annealing can be performed at the same temperature, e.g., 59 to 61°C, for more than 95% of the sets included in the library.

Usually, optimal conditions of dPCR using sets of primers and probes for different amplification targets are often different. If the annealing temperature differs from set to set, the reactions of PCR should be performed with multiple separate runs, which require time and effort, unless a PCR system in which multiple types of conditions can simultaneously be set is used. However, according to the present invention, embodiments thereof, and specific examples example, the annealing temperature can be set to be constant (e.g., 60°C) for the sets included in the library for many cases by adjusting the primer concentration and adding reagents as described above. Exceptionally, for a few % (e.g., about 3 to 5%) of the sets included in the library, the annealing temperature is preferably set to 58°C, 56°C, or the like. Among the specific primers and probes described in this specification, when the primers designed to analyze EGFR c.2573T>G p.L858R and PIK3CA c.3140A>T p.H1047L are used, it is preferable to perform PCR at 58°C (for the specific primer sequences, see the section of Examples).

### [Use of library]

In one embodiment, the library is used in a method for analyzing a mutation relevant to a cancer in a test subject, which comprises the following steps (1) to (3):
(1) the step of preparing the library described above;
(2) the step of selecting a probe and/or primer for detecting one mutation from the prepared library; and
(3) the step of analyzing a mutation in a nucleic acid obtained from the test subject by digital PCR using the selected probe and/or primer.

The analysis method using the library of the present invention may be used, in one embodiment, in a method of assisting prediction of relapse or prognosis of a cancer in a test subject after a treatment of the cancer. The method of assisting refers to a method conducted by a person other than medical practitioners, for example, clinical laboratory technologist, nurse, public health nurse, test subject himself or herself, etc. Such a method specifically comprises the step of preparing at least one kind of probe and/or primer or primer pair from the library of the present invention prepared beforehand, and analyzing a mutation in a nucleic acid obtained from a test subject by using the prepared probe and/or primer or primer pair. To the library that can be used for such a method, all the explanations for the library already described in this specification are applied.

It is considered that the method provided by the present invention is effective for a variety of cancers including alimentary canal cancers such as esophageal cancer, gastric cancer, and colorectal cancer, pancreatic cancer, thyroid cancer, bladder cancer, liver cancer, melanoma, breast cancer, and blood tumors.

According to a preferred embodiment, the method of the present invention can be performed as follows.

A set considered to be appropriate (for example, a set of primer and probe for analyzing a case-specific mutation detected in mutation analysis of primary lesion) is obtained from the library of the present invention prepared beforehand, and after operation thereof is checked as required, ratio of mutated DNA (mutant allele frequency, %) in a sample appropriately extracted from the test subject is monitored. The sample is preferably plasma, and a mutation can be analyzed as ctDNA.

When a test subject with a small amount of tumor cells, for example, a test subject of stage I, is the object, ctDNAs may not be detected before a treatment, after an operation, or in the follow-up period. In a test subject with a certain amount of tumor cells, for example, a test subject of stage II, reduction of ctDNAs detected before a treatment to 0% can be confirmed after an operation. Further, by analyzing presence or absence or amount of ctDNAs in the follow-up including postoperative adjuvant chemotherapy, relapse can be predicted. According to the method of the present invention, reduction of ctDNAs accompanying shrinkage of tumor caused by chemotherapy or radiotherapy can be confirmed in some test subjects. According to the method of the present invention, relapse may be diagnosed at an earlier stage compared with the conventional CT diagnosis.

Analysis of ctDNA is also advantageous in the point that it is considered to realize systemic monitoring of mutated DNAs characteristic to a tumor. Moreover, it makes it easy to perform analysis over a long period of time after a treatment with less burden on patients, and it is expected to enable quick transfer to secondary treatment based on quick monitoring of increase in "tumor burden" after the treatment.

The library of the present invention makes it unnecessary to design and synthesize primers and probes for each test subject for detecting ctDNAs. By using the library of the present invention including highly versatile primers and probes, or primers and probes designed so as to enable non-intermittent detection of mutations, relapse of cancer after a treatment can be diagnosed at an early stage. It also enables it to easily perform individualized post-treatment follow-up of cancer patients.

The library of the present invention can also be used in methods for early detection or diagnosis of cancers.

The present invention will be further precisely explained below with reference to examples. However, the present invention is no way limited by these examples.

### Examples

### [Creation of library]

Among the sets of primers and probes synthesized by using Hypercool Primer & Probe, sets of primers and probes for detecting mutations with higher occurrence rate are shown in the following tables for each gene. The synthesis was entrusted to Nihon Gene Research Laboratories, Inc. (1-5-2, Nakano, Miyagino-ku, Sendai-shi, Japan). In the base sequences indicated in the tables, the portions indicated with lowercase letters are Tm-increasing base insertion sites (modified nucleotide sites). Specifically, a is 2-amino-deoxyadenine, and c is 5-methyl-deoxycytosine. The wild-type probe (Wt Probe) was labeled with HEX, and the mutant probe (Mt Probe) with FAM.

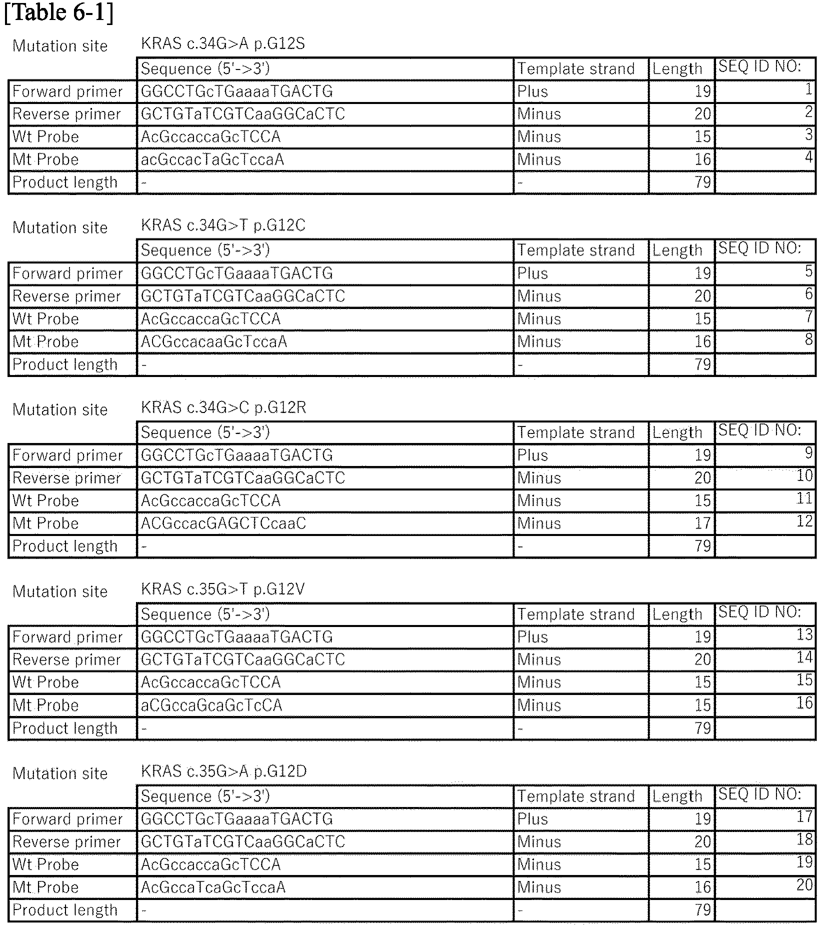

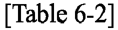

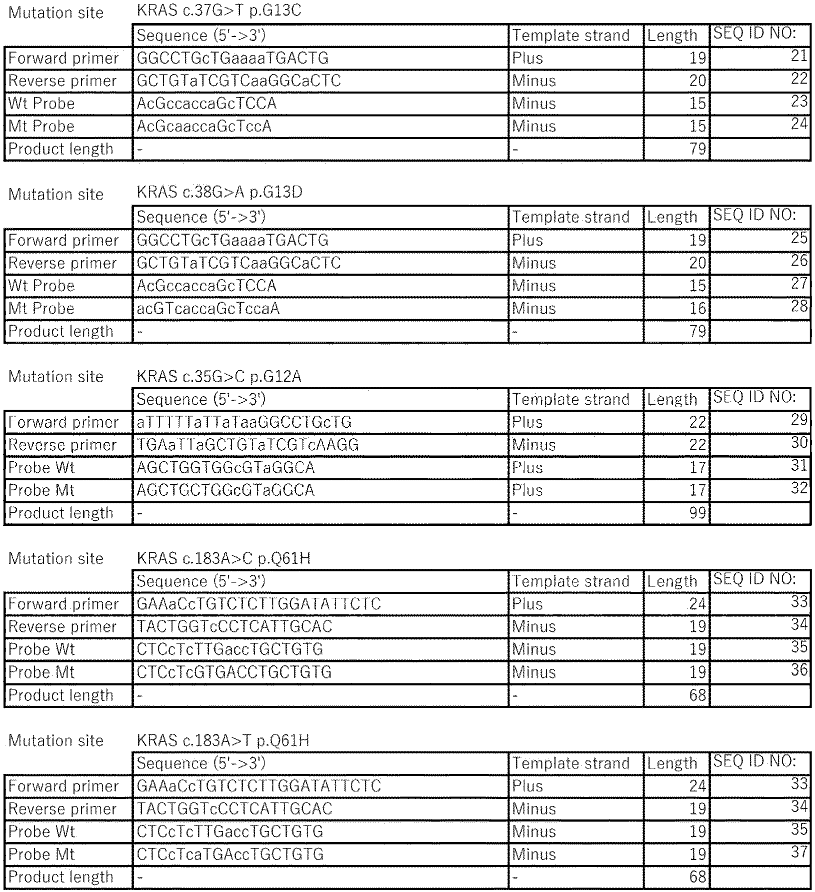

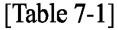

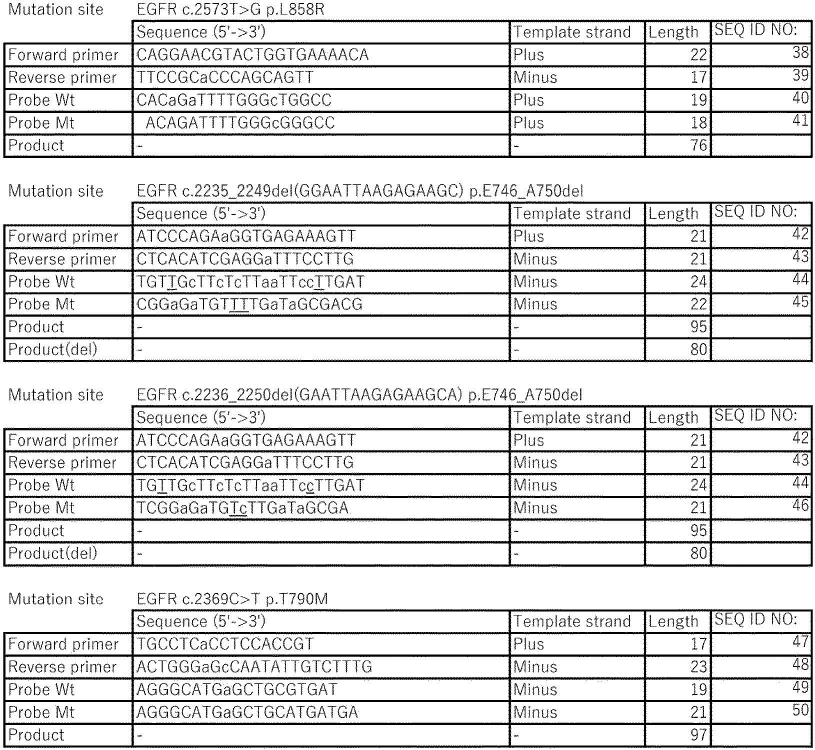

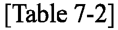

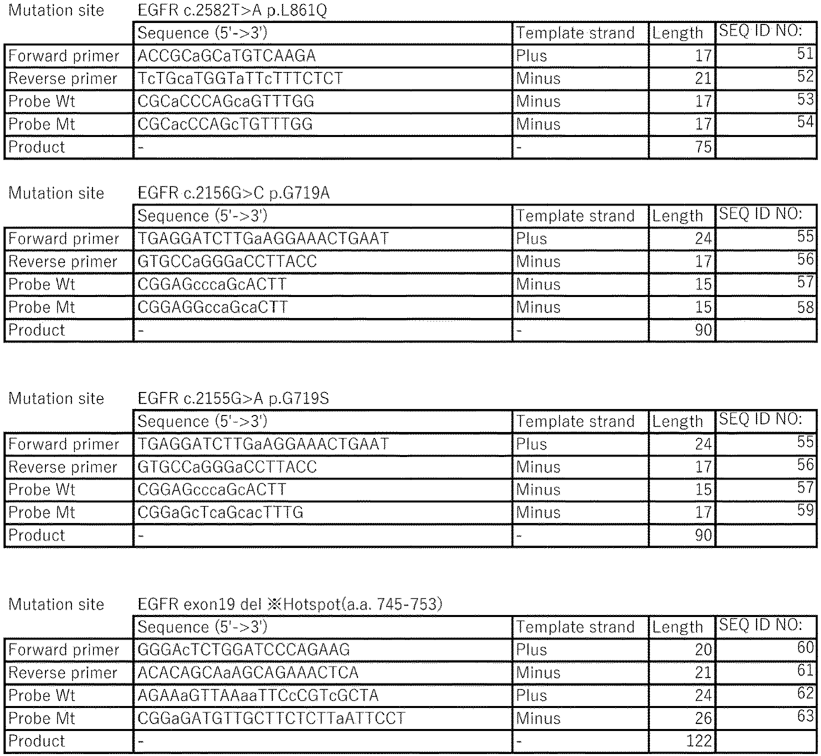

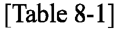

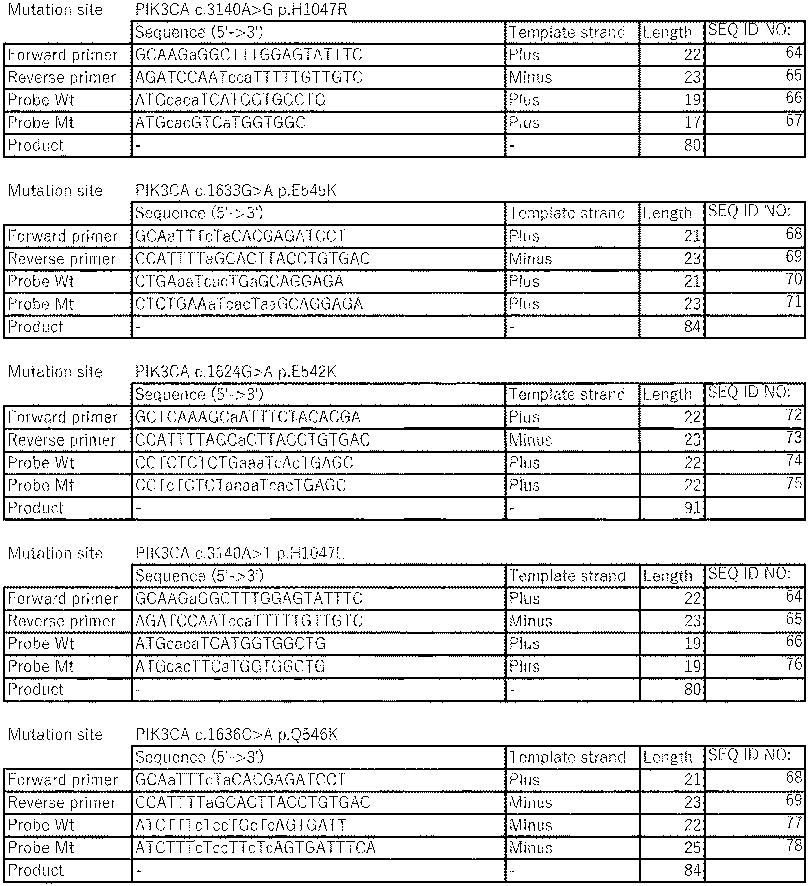

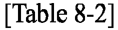

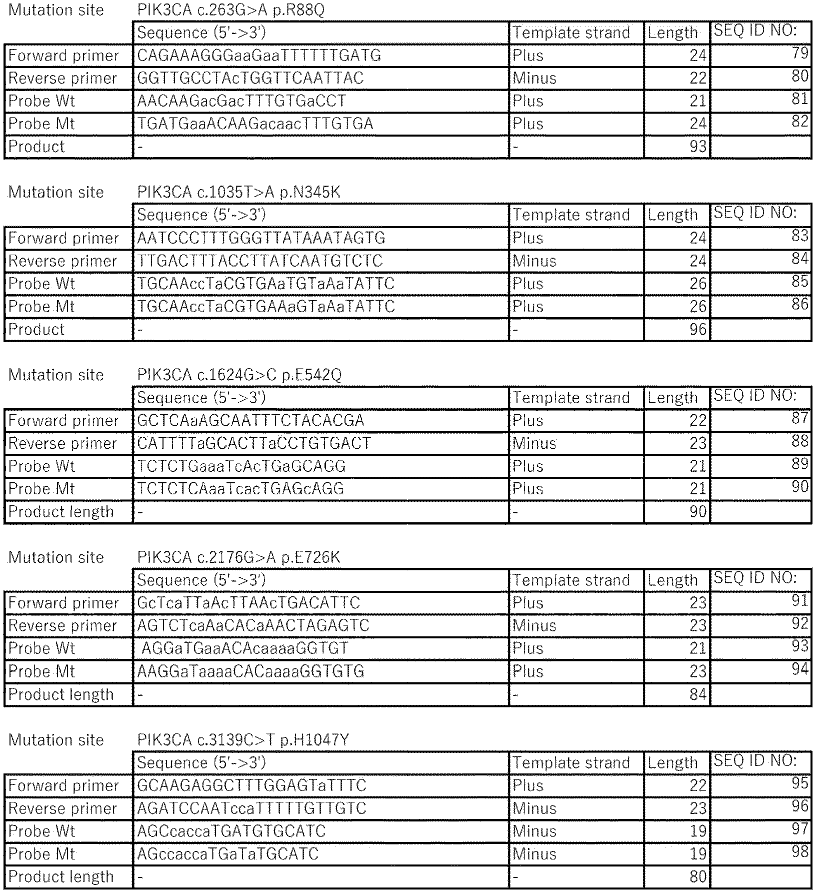

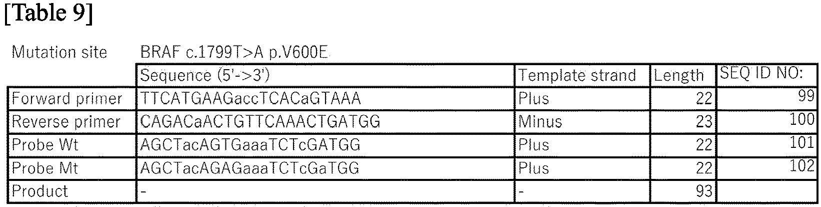

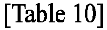

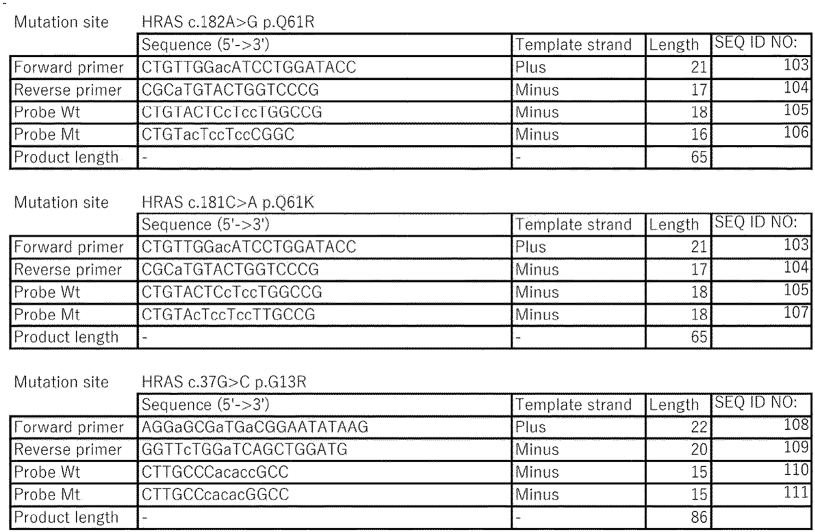

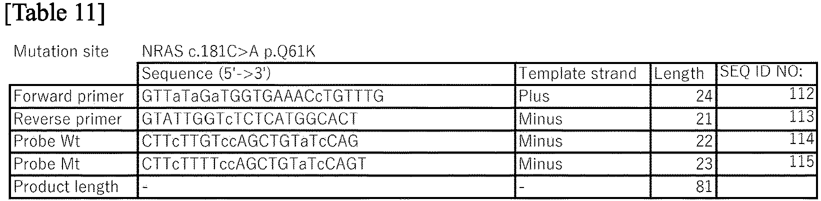

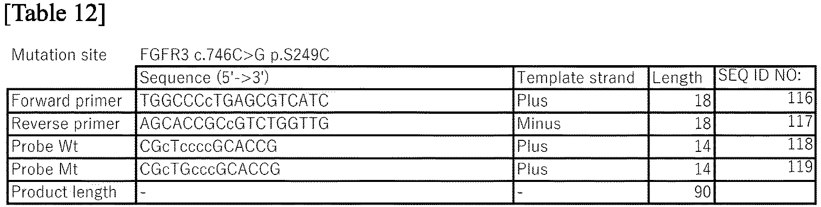

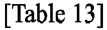

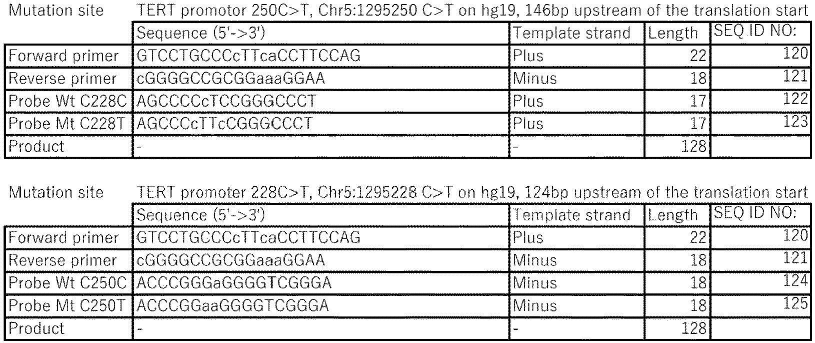

### [Operation check of primer and probe sets]

Operation check of the above sets of primers and probes (36 types) was performed by carrying out dPCR using primary lesion (or sufficient amount of plasma) DNA as the template. Typical PCR conditions for each instrument are described below. In the following experiments, dPCR was performed under one of these conditions, unless especially noted.

PCR conditions used when Thermo Fisher Scientific 3D QuantStudio Digital PCR or BioRad QX200 Droplet Digital PCR system is used:
(1) 95°C, 10 minutes,
(2) 94°C, 30 seconds,
(3) 60°C (58°C or 56°C), 1 minutes, repeat the cycle consisting of (2) to (4) 40 times,
(4) 98°C, 10 minutes, and
(5) 4°C, ∞

PCR conditions used when Thermo Fisher Scientific Absolute Q is used:
(1) 96°C, 10 minutes,
(2) 96°C, 5 seconds,
(3) 60°C (58°C or 56°C), 15 to 45 seconds, and
(4) Repeat the cycle consisting of (2) and (3) about 40 times.

### [Verification of primer concentration]

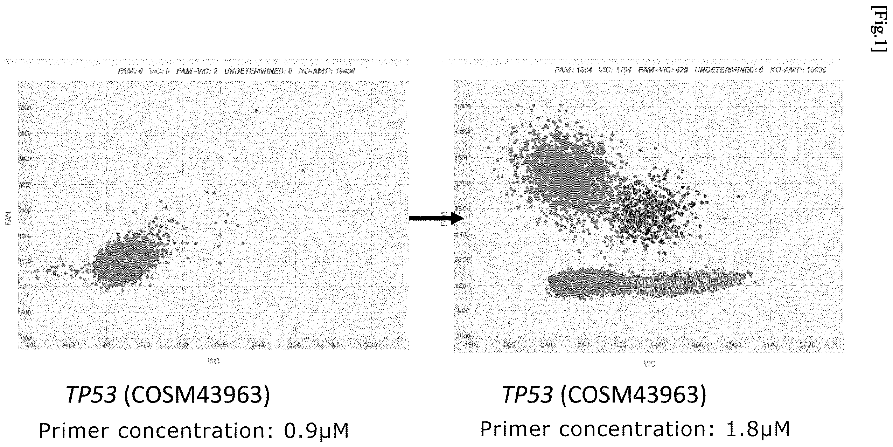

When dPCR was performed with a primer concentration of 0.9 µM for the primers designed and synthesized for detecting the c.396G>C (COSM43963) mutation in TP53, the mutant and wild-type alleles were not separated. When dPCR was performed with a primer concentration (final concentration in the dPCR reaction system) of 0.9 µM, the mutant and wild-type alleles were not separated. However, when dPCR was performed with a primer concentration of 1.8 µM, the mutant allele and the wild-type alleles could be separated (Fig. 1).

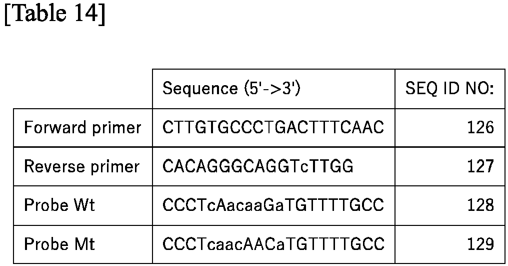

### [Verification of annealing temperature]

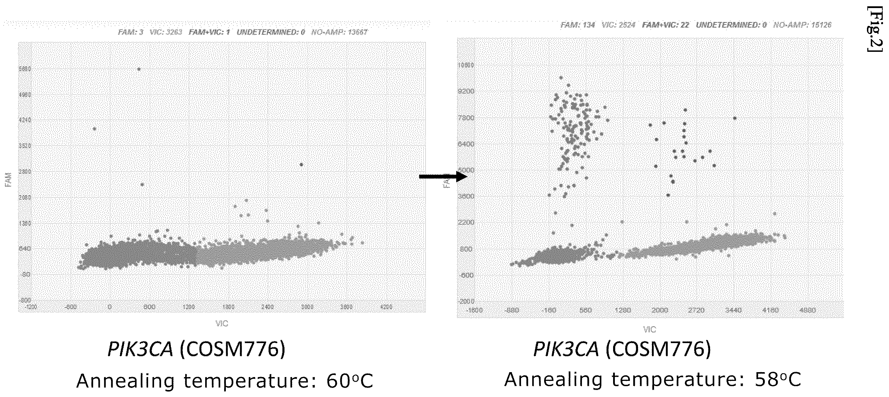

dPCR was performed by using the set of primers and probes for detecting the c.3140A>T (COSM776) mutation in PIC3CA described above (see the above table). When the annealing temperature was set at 58°C, the mutant and wild-type alleles could be separated (Fig. 2).

### [Verification of additives]

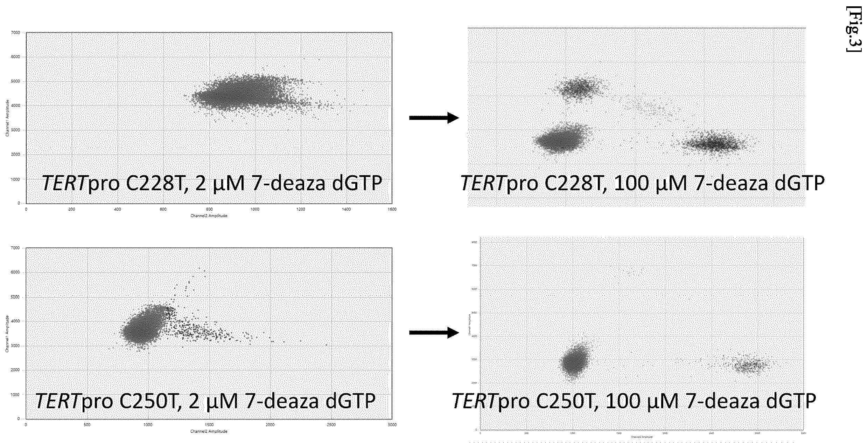

dPCR was performed by using sets of primers and probes for detecting the 228C>T mutation in the TERT promoter and 250C>T mutation in the TERT promoter described above (see the table mentioned above). When dPCR was performed by adding 7-deaza-2'-deoxy-guanosine-5'-triphosphate (7-deaza-dGTP) at a final concentration of 100 µM in the reaction system of dPCR, the mutant and wild-type alleles could be separated (Fig. 3).

### [Example of library design]

The library OTS-50 can be produced by designing and synthesizing sets of primers and probes for detecting the 50 types of mutations mentioned in the following table according to the Hypercool Primer & Probe method. Such a library is useful by itself and also as a subset of a library for detecting more than 1000 types of mutations, including all mutations that are the objects of the detection according to the present invention.

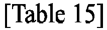

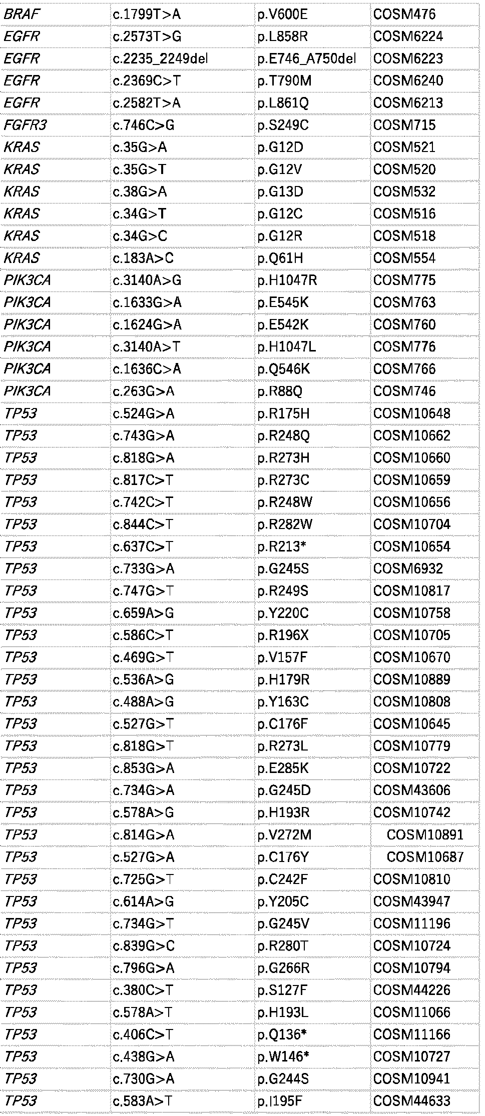

### [Description of the sequences listed in Sequence Listing]

SEQ ID NOS: 1 to 129, sequences of primers or probes for dPCR
SEQ ID NO: 130, ARID1A cDNA
SEQ ID NO: 131, BRAF cDNA
SEQ ID NO: 132, EGFR cDNA
SEQ ID NO: 133, FGFR2 cDNA
SEQ ID NO: 134, FGFR3 cDNA
SEQ ID NO: 135, HRAS cDNA
SEQ ID NO: 136, KRAS cDNA
SEQ ID NO: 137, PIK3CA cDNA
SEQ ID NO: 138, TP53 cDNA
SEQ ID NO: 139, NRAS cDNA
SEQ ID NO: 140, ARID1A gDNA
SEQ ID NO: 141, BRAF gDNA
SEQ ID NO: 142, EGFR gDNA
SEQ ID NO: 143, FGFR2 gDNA
SEQ ID NO: 144, FGFR3 gDNA
SEQ ID NO: 145, HRAS gDNA
SEQ ID NO: 146, KRAS gDNA
SEQ ID NO: 147, PIK3CA gDNA
SEQ ID NO: 148, TP53 gDNA
SEQ ID NO: 149, NRAS gDNA
SEQ ID NO: 150, TERT_promoter_region

## Claims

1. A library constituted by a plurality of probes and/or primers for detecting a mutation relevant to a cancer in a gene selected from the group consisting of ARID1A, BRAF, EGFR, FGFR2, FGFR3, HRAS, KRAS, and PIK3CA or a promoter region thereof, wherein the plurality of probes and/or primers include those for detecting:
c.5965C>T, c.5548dup, and c.3826C>T for ARID1A;
c.1799T>A for BRAF;
c.2573T>G, c.2235_2249del, and c.2236_2250del for EGFR;
c.755C>G, c.1650T>A, and c.1147T>C for FGFR2;
c.746C>G, c.1124A>G, and c.742C>T for FGFR3;
c.37G>C, c.182A>G, c.35G>T, c.182A>T, c.181C>A, c.34G>A, and c.35G>A for HRAS;
c.35G>A, c.35G>T, c.38G>A, and c.34G>T for KRAS; and
c.3140A>G, c.1633G>A, and c.1624G>A for PIK3CA.

2. The library according to claim 1, which further comprises probes and/or primers for detecting:
c.5161C>T, c.2077C>T, c.6259G>A, c.4003C>T, c.4336C>T, c.5164C>T, and c.6472C>T for ARID1A,
c.1798_1799delinsAA, c.1801A>G, c.1406G>C, and c.1798G>A for BRAF, c.2369C>T, c.2240 _2257del, c.2240_2254del, c.2582T>A, c.866C>T, c.2156G>C, and c.1793G>T for EGFR,
c.1127A>G, c.557T>C, c.1650T>G, c.696A>G, c.1978A>G, c.758C>G, and c.870G>C for FGFR2,
c.1114G>T, c.1954A>G, c.1117A>T, c.417C>T, c.1955A>T, c.2095G>T, and c.1178C>A for FGFR3,
c.38G>T, c.38G>A, and c.34G>T for HRAS,
c.35G>C, c.34G>A, c.34G>C, c.37G>T, c.183A>C, and c.182A>T for KRAS, and
c.3140A>T, c.263G>A, c.1035T>A, c.1636C>A, c.1258T>C, c.1634A>G, and c.2176G>A for PIK3CA.

3. The library according to claim 1, which is for detecting a mutation relevant to a cancer by digital PCR, and wherein the primers are used at a final concentration of 450 to 3600 nM.

4. The library according to claim 1, which is for detecting a mutation relevant to a cancer by digital PCR, and wherein the digital PCR is performed in the presence of at least one of 20 to 500 µM 7-deaza-2'-deoxy-guanosine-5'-triphosphate and 0.15 to 3.8% dimethyl sulfoxide, as the case may be.

5. The library according to claim 1, which is for detecting a mutation relevant to a cancer by digital PCR, and wherein annealing is performed at 55 to 61°C.

6. The library according to any one of claims 1 to 5, wherein the probes and/or primers are oligonucleotides that may be labeled, each of which consists of any one of the sequences of SEQ ID NOS: 1 to 144 that may contain a modified base.

7. A method for analyzing a mutation relevant to a cancer in a gene selected from the group consisting of ARID1A, BRAF, EGFR, FGFR2, FGFR3, HRAS, KRAS, and PIK3CA in a test subject, the method comprising the following steps (1) to (3):
(1) preparing a library constituted by a plurality of probes and/or primers, wherein the plurality of probes and/or primers include those for detecting:
c.5965C>T, c.5548dup, and c.3826C>T for ARID1A;
c.1799T>A for BRAF;
c.2573T>G, c.2235_2249del, and c.2236_2250del for EGFR;
c.755C>G, c.1650T>A, and c.1147T>C for FGFR2;
c.746C>G, c.1124A>G, and c.742C>T for FGFR3;
c.37G>C, c.182A>G, c.35G>T, c.182A>T, c.181C>A, c.34G>A, and c.35G>A for HRAS;
c.35G>A, c.35G>T, c.38G>A, and c.34G>T for KRAS; and
c.3140A>G, c.1633G>A, and c.1624G>A for PIK3CA.
(2) selecting a set of probes and/or primers for detecting one mutation from the prepared library; and
(3) analyzing a mutation in a nucleic acid obtained from the test subject by digital PCR using the selected set of probes and/or primers.

8. The method according to claim 7, wherein the primers are used at a final concentration of 450 to 3600 nM.

9. The method according to claim 7, wherein the digital PCR is performed in the presence of at least one of 20 to 500 nM 7-deaza-2'-deoxy-guanosine-5'-triphosphate and 0.15 to 3.8% dimethyl sulfoxide, as the case may be.

10. The method according to claim 7, wherein annealing is performed at 55 to 61°C.

11. The method according to any one of claims 7 to 10, wherein the probes and/or primers are oligonucleotides that may be labeled, each of which consists of any one of the sequences of SEQ ID NOS: 1 to 137 that may contain a modified base.

12. A probe or primer consisting of any of the following sequences (lowercase a is 2-amino-deoxyadenine and lowercase c is 5-methyl-deoxycytosine), which is for detecting a mutation relevant to a cancer by digital PCR.

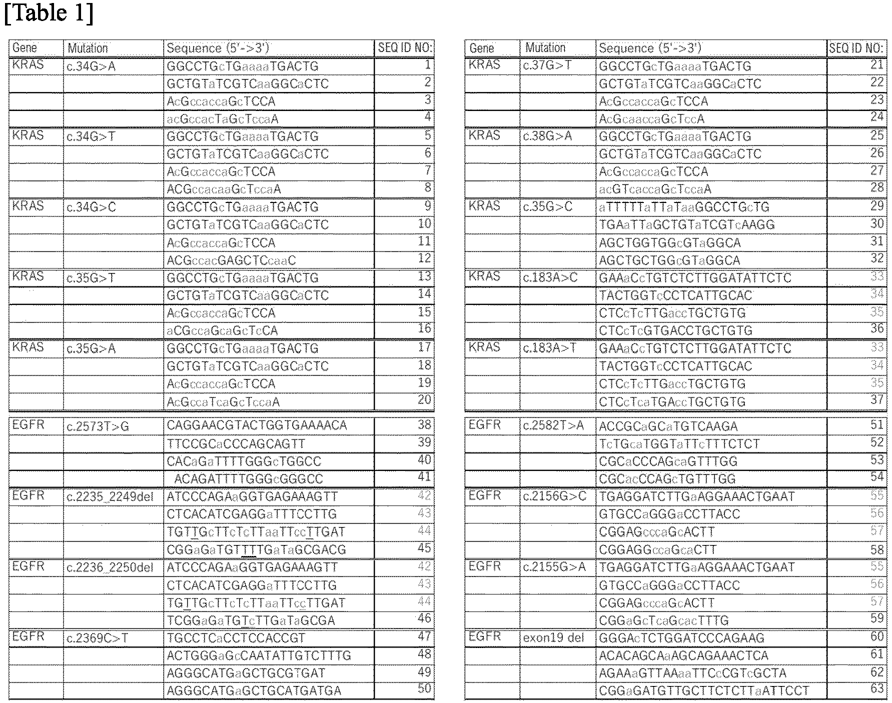

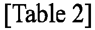

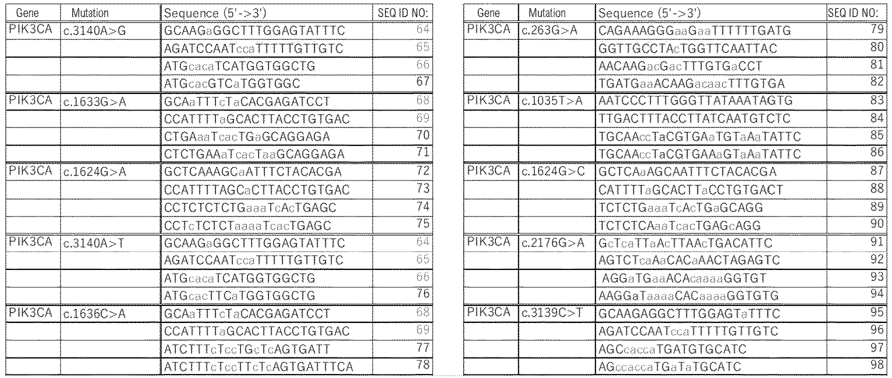

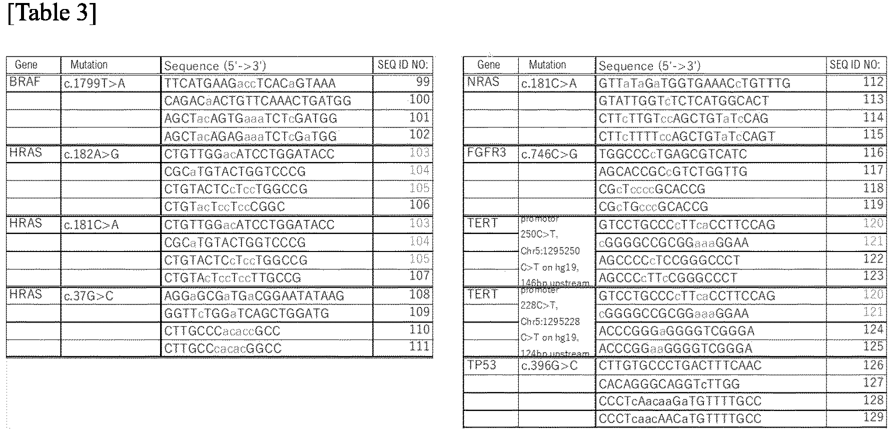

13. A library for use in digital PCR comprising at least one set of oligonucleotides selected from the group consisting of the following sets of oligonucleotides, wherein sequences of the oligonucleotides may contain a modified nucleotide, and the oligonucleotides may be labeled:
oligonucleotides consisting of the sequences of SEQ ID NOS: 1 to 4,
oligonucleotides consisting of the sequences of SEQ ID NOS: 5 to 8,
oligonucleotides consisting of the sequences of SEQ ID NOS: 9 to 12,
oligonucleotides consisting of the sequences of SEQ ID NOS: 13 to 16,
oligonucleotides consisting of the sequences of SEQ ID NOS: 17 to 20,
oligonucleotides consisting of the sequences of SEQ ID NOS: 21 to 24,
oligonucleotides consisting of the sequences of SEQ ID NOS: 25 to 28,
oligonucleotides consisting of the sequences of SEQ ID NOS: 29 to 32,
oligonucleotides consisting of the sequences of SEQ ID NOS: 33 to 36,
oligonucleotides consisting of the sequences of SEQ ID NOS: 33 to 35 and 37,
oligonucleotides consisting of the sequences of SEQ ID NOS: 38 to 41,
oligonucleotides consisting of the sequences of SEQ ID NOS: 42 to 45,
oligonucleotides consisting of the sequences of SEQ ID NOS: 42 to 44 and 46,
oligonucleotides consisting of the sequences of SEQ ID NOS: 47 to 50,
oligonucleotides consisting of the sequences of SEQ ID NOS: 51 to 54,
oligonucleotides consisting of the sequences of SEQ ID NOS: 55 to 59,
oligonucleotides consisting of the sequences of SEQ ID NOS: 60 to 63,
oligonucleotides consisting of the sequences of SEQ ID NOS: 64 to 67,
oligonucleotides consisting of the sequences of SEQ ID NOS: 68 to 71,
oligonucleotides consisting of the sequences of SEQ ID NOS: 72 to 75,
oligonucleotides consisting of the sequences of SEQ ID NOS: 64 to 66, and 76,
oligonucleotides consisting of the sequences of SEQ ID NOS: 68, 69, 77, and 78,
oligonucleotides consisting of the sequences of SEQ ID NOS: 79 to 82,
oligonucleotides consisting of the sequences of SEQ ID NOS: 83 to 86,
oligonucleotides consisting of the sequences of SEQ ID NOS: 87 to 90,
oligonucleotides consisting of the sequences of SEQ ID NOS: 91 to 94,
oligonucleotides consisting of the sequences of SEQ ID NOS: 95 to 98,
oligonucleotides consisting of the sequences of SEQ ID NOS: 99 to 102,
oligonucleotides consisting of the sequences of SEQ ID NOS: 103 to 106,
oligonucleotides consisting of the sequences of SEQ ID NOS: 103 to 105, and 107,
oligonucleotides consisting of the sequences of SEQ ID NOS: 108 to 111,
oligonucleotides consisting of the sequences of SEQ ID NOS: 112 to 115,
oligonucleotides consisting of the sequences of SEQ ID NOS: 116 to 119,
oligonucleotides consisting of the sequences of SEQ ID NOS: 120 to 123,
oligonucleotides consisting of the sequences of SEQ ID NOS: 120, 121, 124, and 125, and
oligonucleotides consisting of the sequences of SEQ ID NOS: 126 to 129.
